# EUROPÄISCHE PATENTSCHRIFT

(11) **EP 1 438 017 B1**
(45) Veröffentlichungstag und Bekanntmachung des Hinweises auf die Patenterteilung: **10.05.2006**
(21) Anmeldenummer: 02787493.2
(22) Anmeldetag: 18.10.2002
(51) Int. Cl.: A61K 8/02, A61K 8/34, A61K 8/60, A61K 8/37, A61Q 19/10

(54) **IMPRÄGNIERLÖSUNG FÜR KOSMETIKTÜCHER**
IMPREGNATING SOLUTION FOR COSMETIC CLOTHS
SOLUTION D'IMPREGNATION POUR SERVIETTES A USAGE COSMETIQUE

(30) Priorität: 26.10.2001 DE 10152942; 18.12.2001 DE 10162184
(43) Veröffentlichungstag der Anmeldung: 21.07.2004
(73) Patentinhaber: Cognis IP Management GmbH, 40589 Düsseldorf (DE)
(72) Erfinder: HENSEN, Hermann, 42781 Haan (DE); LAZAROWITZ, Virginia, Hatfield, PA 19440 (US); MORRIS, Timothy, Morton, PA 19070 (US); KUBLIK, Heike, 47906 Kempen (DE); SEARLE, Jackie, Hawley/Near Datford, Kent DA2 7RU (GB); LEONARD, Mark, Bexley, Kent DA5 1AZ (GB)
(86) Internationale Anmeldenummer: PCT/EP2002/011674
(87) Internationale Veröffentlichungsnummer: WO 2003/037293

(56) Entgegenhaltungen:
- EP-A- 0 366 070
- WO-A-01/79418
- WO-A-99/13861

## Beschreibung

### Gebiet der Erfindung

Die Erfindung befindet sich auf dem Gebiet der Kosmetik und betrifft Imprägnierlösungen, die definierte Pflege- und Reinigungsstoffe mit partikulären Wachskörpern enthalten, für die Anwendung auf Kosmetiktüchem mit einer besonderen Struktur.

### Stand der Technik

In zunehmendem Maße gewinnen Kosmetiktücher als eine einfache und hygienische Anwendungsform, die dem Zeitgeist der heutigen Körperpflege entgegenkommt, an Bedeutung. Zwei Formen dieser Anwendungen sind bereits von unterschiedlichen Anbietern im Handel. Feuchttücher, die textile Gewebe, Vliese oder aber auch Tissuepapiere darstellen, welche mit einer reinigenden oder pflegenden Formulierung getränkt sind und trockene Kosmetiktücher, die vor der Anwendung befeuchtet werden müssen.
Zahlreiche Patentanmeldungen beschreiben Reinigungstücher, bei denen Lösungen auf unterschiedliche Gewebe aufgezogen werden. Ausgehend von der bei den ersten marktreifen Kosmetiktüchem angestrebten Reinigungsfunktion steht heute immer mehr die Pflege im Mittelpunkt. So werden beispielsweise in der internationalen Patentanmeldung **WO 95/35411** Feuchttücher vorgeschlagen, welche mit einer Lotion imprägniert sind, die neben Mineralöl, Fettsäureester, Fettalkoholethoxylate und Fettalkohole enthalten.
Für trockene Kosmetiktücher seien stellvertretend die Patentanmeldungen **WO 99/13861** und **WO 01/08657** genannt.

WO 01/79418 offenbart trockene Kosmetiktüchern, die Alkyldiole und schäumende Tenside enthalten. Tabelle 1 zeigt Imprägnierlösungen für die Kosmetiktücher, die u.a. Kationpolymere (Polyquaternium 7), amphoterische Tenside (Cocamidopropyl Betaine) und nonionische Tenside (Decyl Polyglucoside) enthalten.

Dabei werden in der Regel strukturlose, nicht gewebte Stoffe mit tensid- und pflegestoffhaltigen Formulierungen imprägniert und/oder überzogen und gegebenenfalls nachfolgend getrocknet. Je nach Größe des Trägers kann es sich hierbei um imprägnierte Einmalwaschlappen oder auch kleinere Reinigungspads handeln.
Die Anforderungen an den Reinigungseffekt, das Hautgefühl während und nach der Verwendung und die Anwendungsfreundlichkeit stellen eine technische Herausforderung für den Entwickler dar. So hat die nach dem Anfeuchten oder bereits in den befeuchteten Tüchern generierte Schaummenge, -stabilität und Schaumstruktur einen wesentlichen Einfluß auf das sensorische Empfinden während und nach der Reinigung. Die geeignete Schaumstruktur und ein ausreichendes Schaumvolumen nach dem Befeuchten sind ohne starke mechanische Bearbeitung des Tuches mit den Händen schwer zu erreichen. Durch weitere pflegende nichttensidische Zusätze kann dieses Problem zunehmend erschwert werden.

Es ist daher Aufgabe der vorliegenden Erfindung Formulierungen zur Verfügung zu stellen, zur Imprägnierung von feuchten und trockenen Kosmetiktüchern mit verbesserten Reinigungs-, pflegenden und sensorischen Eigenschaften für die Pflege und Reinigung von Körper und Haaren.
Es ist dabei besonders eine porentiefe Reinigung sowie ein angenehmes Hautgefühl während und nach der Anwendung angestrebt, das durch die optimierten Schaumeigenschaften des Mittels unterstützt werden soll.

### Beschreibung der Erfindung

Gegenstand der Erfindung sind Zubereitungen zur Imprägnierung von Kosmetiktüchern, **dadurch gekennzeichnet, dass** sie
a) eine Emulgatormischung, enthaltend nichtionische und amphotere Tenside im Mengenverhältnis 10 : 1 bis 1 : 1 bezogen auf die Menge der Emulgatoren und
b) eine Mischung aus Wachskörpern, enthaltend Wachsester, Partialglyceride und Fettalkoholethoxylate sowie
c) mindestens ein Kationpolymer
enthalten.

Es wurde gefunden, dass Formulierungen der angegebenen Zusammensetzung auf einer strukturierten Oberfläche eines Trägers (Kosmetikiuch) zu einer vorteilhaften Schaumbildung, einem gleichzeitig guten Reinigungseffekt und einem angenehmen Hautgefühl führen. Durch die Kombination des Trägers mit Imprägnierlösungen mit pflegenden, feindispergierten partikulären Wachsdispersionen definierter Teilchengröße kann durch geringen mechanischen Einfluß schnell ein feinporiger voluminöser Schaum generiert werden. Es hat sich dabei insbesondere die Zusammensetzung von Wachkörpem, enthaltend Wachsester, Partialglyceride und Fettalkoholethoxylaten bewährt, sowie eine Emulgatormischung aus nichtionischen und amphoteren Tensiden im Mengenverhältnis 10 : 1 bis 1 : 1.
Imprägnierlösungen dieser Zusammensetzung zeigten ein besonders gutes Hautgefühl während und nach der Anwendung der damit imprägnierten Kosmetiktücher.
Zusätzlich hat die Größe der Wachspartikel einen Einfluß auf die Schaumeigenschaften und das Hautgefühl während und nach der Reinigung. Je kleiner die Partikel vorliegen, desto angenehmer ist die Sensorik.

### Träger für Kosmetiktücher

Die erfindungsgemäßen Imprägnierlösungen sind geeignet zur Anwendung auf feuchten Kosmetiktüchern, vorzugsweise werden sie jedoch auf trockenen Kosmetiktüchern eingesetzt.

Die speziellen Trägersysteme, auf die sich die vorliegende Erfindung bezieht, können ein- oder mehrlagig aufgebaut sein. Neben papierbasierten Tissues kommen auch entsprechende Tissuegewebe in Frage, die aus Faser- oder Fleecestoff hergestellt werden. Zu Beispielen für Naturfasern zählen Seide, Cellulose, Keratin, Wolle, Baumwolle, Jute, Leinen, Flaks, für synthetische Fasern Acetat-, Acrylat-, Celluloseester-, Polyamid-, Polyester-, Polyolefin-, Polyvinylalkohol-, Polyurethan- Fasern, oder auch durch Additive hydrophilierte Polyolefingewebe sowie Mischungen dieser Fasern bzw. Gewebe. Umsetzungsprodukte von 1 Teil Polyethylenglykol mit 2 Teilen Fettsäuren mit 10 bis 12 C-Atomen oder deren Derivaten werden hierbei zur Hydrophilierung der Polyolefin-haltigen Gewebe eingesetzt.
Bevorzugt sind nicht-gewebte Stoffe, da diese besser mit der erfindungsgemäßen erwünschten Struktur versehen werden können, dazu eignen sich Träger aus Viskose- Polyester-Mischungen besonders. Bevorzugt sind jedoch durch Wassereinwirkung vernetzte Trägersysteme von 50 bis 90 Gew. % Viskose und 50 bis 10 Gew. % Polyester, besonders bevorzugt sind Träger aus 60 bis 80 Gew. % Viskose und 40 bis 20 Gew. % Polyester, speziell werden Stoffe mit 65 bis 70 Gew. % Viskose und 35 bis 30 Gew. % Polyester eingesetzt.

Die Größe der Tücher liegt in der Regel zwischen 100 und 500 mm in der Länge und zwischen 100 und 500 mm in der Breite, wobei Längen- und Breitenmaße zwischen 120 und 220 mm bevorzugt sind. Das Gewebe kann aber auch in Handschuhform vorliegen und ist dann möglicherweise mehrlagig, so dass die innere Gewebelage des Handschuhs hydrophober ausfällt, eine Barrierefunktion hat und einen Schutz vor der Berührung der Hand mit der Formulierung oder mit Feuchtigkeit bietet.

Die Trägerstoffe der erfindungsgemäßen Kosmetiktücher weisen auf Grund ihrer Herstellung - der Wassereinwirkung mit einem sogenannten Hydroentanglement Belt - eine gleichmäßig, strukturierte Oberfläche mit punktförmigen bis ovalen Vertiefungen auf. Diese Vertiefungen - auch Grübchen genannt - haben eine runde bis ovale Form mit 0,1 bis 1 mm, vorzugsweise 0,2 bis 0,6 mm Durchmesser resp. Breite und 0,5 bis 5,0 mm, vorzugsweise 0,8 bis 1,5 mm Durchmesser resp. Länge. Sie können beidseitig vorliegen oder nur von einer Seite. Bei einseitigen Vertiefungen nehmen sie zwischen 50 und 99 %, vorzugsweise zwischen 60 und 85 % der Dicke des Trägers ein, bei beidseitigen Vertiefungen muß dieser Anteil entsprechend aufgeteilt werden. Pro 100 mm² Trägerfläche liegen im Durchschnitt zwischen 500 und 4000, vorzugsweise zwischen 1500 und 3500, und besonders bevorzugt zwischen 2500 und 3200 solcher Grübchen vor.

### Imprägnierlösungen

Unter der Bezeichnung Imprägnierlösung - auch Coatinglösungen genannt - sind die erfindungsgemäßen Zubereitungen in Form von Lösungen, Dispersionen und Emulsionen zu verstehen, die auf die Trägerstoffe für Kosmetiktücher aufgetragen werden.

Das Gewichtsverhältnis vom trockenen Gewebe zu aufgebrachter Reinigungs- und Pflegelösung soll 60:40 bis 90:10 und vorzugsweise 85:15 bis 80:20 betragen. Die Imprägnierlösung enthält Tenside und Wachsdispersionen mit durchschnittlichen Partikelgrößen von bis maximal 13 µm, bevorzugt maximal 4 µm und besonders bevorzugt maximal 2 µm.

### Emulgatormischungen

In der Emulgatormischung, deren Gesamtanteil an den Imprägnier- oder Coatinglösungen üblicherweise etwa 1,5 bis 75, vorzugsweise 15 bis 55 und besonders bevorzugt 25 bis 40 Gew.-% beträgt sind nichtionische, amphotere und gegebenenfalls anionische Tenside enthalten. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217 verwiesen.

Typische Beispiele für besonders geeignete milde, d.h. besonders hautverträgliche Tenside sind Fettalkoholpolyglycolethersulfate, Monoglyceridsulfate, Mono- und/oder Dialkyl-sulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetate und/oder Proteinfettsäurekondensate, letztere vorzugsweise auf Basis von Weizenproteinen.
In der vorliegenden Erfindung bevorzugte Tenside sind Dinatrium Cocoamphodiacetat, Natrium Cocoamphoacetat, Cocamidopropyl Betain, Cocamide DEA, Alkyloligoglucoside und deren Mischungen.
Besonders bevorzugt sind nichtionische Tenside ausgewählt aus der Gruppe, die gebildet wird von Alkyloligoglucosiden, Cocamidopropyl Betain, PEG-7 Glyceryl Cocoat, Laureth-4, Ceteareth-12, Ceteareth-20 und/oder Beheneth-10 und speziell bevorzugt sind Tensidmischungen aus Alkyloligoglycosiden und Betainen, insbesondere Cocamidopropylbetain, die im Mengenverhältnis 10 : 1 bis 1 : 1, vorzugsweise 5 : 1 bis 1,5 : 1 und besonders bevorzugt 4 : 1 bis 2 : 1 vorliegen.

**Alkyl- und Alkenyloligoglykoside** stellen bekannte nichtionische Tenside dar, die der Formel **(I)** folgen,

**R**^{**1**}**O-[G]**_{**p**} **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlä-gigen Verfahren der präparativen organischen Chemie erhalten werden. Stellvertretend für das umfangreiche Schrifttum sei hier auf die Übersichtsarbeit von Biermann et al. in **Starch/Stärke 45, 281 (1993),** B.Salka in **Cosm.Toil. 108, 89 (1993)** sowie J.Kahre et al. in **SÖFW-Journal Heft 8, 598 (1995)** verwiesen.

Die Alkyl- und/oder Alkenyloligoglykoside können sich von Aldosen bzw. Ketosen mit 5 oder 6 Kohlen-stoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligo-glykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligo-glucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidyl-alkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden kön-nen. Bevorzugt sind Alkyloligoglucoside auf Basis von gehärtetem C_{12/14-}Kokosalkohol mit einem DP von 1 bis 3.

**Betaine** stellen bekannte Tenside dar, die überwiegend durch Carboxyalkylierung, vorzugsweise Carb-oxymethylierung von aminischen Verbindungen hergestellt werden. Vorzugsweise werden die Aus-gangsstoffe mit Halogencarbonsäuren oder deren Salzen, insbesondere mit Natriumchloracetat kon-densiert, wobei pro Mol Betain ein Mol Salz gebildet wird. Ferner ist auch die Anlagerung von unge-sättigten Carbonsäuren, wie beispielsweise Acrylsäure möglich. Zur Nomenklatur und insbesondere zur Unterscheidung zwischen Betainen und "echten" Amphotensiden sei auf den Beitrag von U.Ploog in **Seifen-Öle-Fette-Wachse, 108, 373 (1982)** verwiesen. Weitere Übersichten zu diesem Thema finden sich beispielsweise von A.O'Lennick et al. in **HAPPI, Nov. 70 (1986),** S.Holzman et al. in **Tens. Surf.Det. 23 309 (1986)**, R.Bibo et al. in Soap Cosm.Chem.Spec., **Apr. 46 (1990)** und P.Ellis et al. in **Euro Cosm. 1, 14 (1994).** Beispiele für geeignete Betaine stellen die Carboxyalkylierungsprodukte von sekundären und insbesondere tertiären Aminen dar, die der Formel **(II)** folgen, 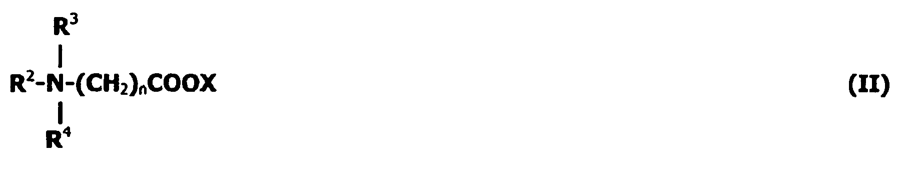 in der R² für Alkyl- und/oder Alkenylreste mit 6 bis 22 Kohlenstoffatomen, R³ für Wasserstoff oder Alkylreste mit 1 bis 4 Kohlenstoffatomen, R⁴ für Alkylreste mit 1 bis 4 Kohlenstoffatomen, n für Zahlen von 1 bis 6 und X für ein Alkali- und/oder Erdalkalimetall oder Ammonium steht. Typische Beispiele sind die Carboxymethylierungsprodukte von Hexylmethylamin, Hexyldimethylamin, Octyldimethylamin, De-cyldimethylamin, Dodecylmethylamin, Dodecyldimethylamin, Dodecylethylmethytamin, C_{12/14}-Kokosalkyldimethylamin, Myristyldimethylamin, Cetyldimethylamin, Stearyldimethylamin, Stearylethylmethyl-amin, Oleyldimethylamin, C_{16/18}-Talgalkyldimethylamin sowie deren technische Gemische.

Weiterhin kommen auch Carboxyalkylierungsprodukte von Amidoaminen in Betracht, die der Formel **(III)** folgen, 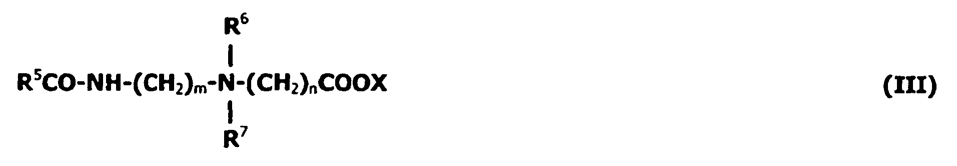 in der R⁵CO für einen aliphatischen Acylrest mit 6 bis 22 Kohlenstoffatomen und 0 oder 1 bis 3 Doppelbindungen, m für Zahlen von 1 bis 3 steht und R⁶, R⁷, n und X die oben angegebenen Bedeutungen haben. Typische Beispiele sind Umsetzungsprodukte von Fettsäuren mit 6 bis 22 Kohlen-stoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linol-säure, Linolensäure, Elaeostearinsäure, Arachinsäure, Gadoleinsäure, Behensäure und Erucasäure so-wie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Bevorzugt ist der Einsatz eines Kondensationsproduktes von C_{8/18}-Kokosfettsäure-N,N-dimethylaminopropylamid mit Natriumchloracetat.

Neben den obligatorisch vorhandenen nichtionischen und amphoteren Tensiden können in den erfindungsgemäßen Imprägnierlösungen weitere Tenside wie anionische oder kationische Tenside vorhanden sein. Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)-sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acyl-aspartate, Alkyloligoglucosidsulfate, Proteinfettsäurekondensate (insbesondere pflanzliche Produkte auf Weizenbasis) und Alkyl(ether)-phosphate. Sofern die anionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminestersalze.

Von den fakultativ einzusetzenden weiteren Tensiden werden bevorzugt die anionischen Tenside (Komponente e))verwendet. Diese werden im Verhältnis zu den amphoteren Tensiden im Mengenverhältnis 0 bis 1 : 1, vorzugsweise 0,3 bis 0,6 : 1 bezogen auf die amphoteren Tenside eingesetzt.

### Wachsdispersionen

Der Anteil der in den Zubereitungen enthaltenen Wachse in den erfindungsgemäßen Imprägnier- und Coatinglösungen beträgt 0,2 bis 30 Gew. %, vorzugsweise 1 bis 25 Gew.% und besonders bevorzugt 2 bis 20 Gew. % bezogen auf die Imprägnierlösungen.

In den erfindungsgemäßen Wachsmischungen sind Wachsester, Partialglyceride und Fettalkoholethoxylate enthalten. Als weitere Wachskörper können Substanzen aus den Gruppen der Alkylenglycolester, Fettsäurealkanolamide, Triglyceride, Ester von mehrwertigen und/oder einwertigen, gegebenenfalls hydroxysubstituierten Carbonsäuren, Fettalkohole, Fettketone, Fettsäuren, Fettaldehyde, Fettether, Fettcarbonate, Ringöffnungsprodukte von Olefinepoxiden sowie deren Mischungen enthalten sein.

Bei den **Wachsestern** handelt es sich üblicherweise um **Ester von einwertigen und mehrwertigen, verzweigten und unverzweigten, gesättigten und ungesättigten, gegebenenfalls hydroxysubstituierten Carbonsäuren** mit Fettalkoholen mit 6 bis 22 Kohlenstoffatomen. Als Säurekomponente dieser Ester kommen beispielsweise Palmitinsäure, Palmoleinsäure, Stearinsäure, Isostearinsäure, Ölsäure, Malonsäure, Maleinsäure, Fumarsäure, Adipinsäure, Sebacinsäure, Azelainsäure, Dodecandisäure, Phthalsäure, Isophthatsäure , Bernsteinsäure sowie Äpfelsäure, Citronensäure und insbesondere Weinsäure und deren Mischungen in Betracht. Die Fettalkohole enthalten 6 bis 22, vorzugsweise 12 bis 18 und insbesondere 16 bis 18 Kohlenstoffatome in der Alkylkette . Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Die Ester können als Voll oder Partialester vorliegen. Bevorzugt ist der Einsatz von Estern linearer gesättigter Fettsäuren mit Cetylalkohol, speziell Cetylpalmitat.

**Partialglyceride,** stellen Mono und/oder Diester des Glycerins mit linearen und verzweigten, gesättigten und ungesättigten Fettsäuren, nämlich beispielsweise Capronsäure, Caprylsäure, Caprinsäure, Laurinsäure, Myristinsäure, Palmitinsäure, Palmoleinsäure, Stearinsäure, Isostearinsäure, Behensäure sowie deren technische Mischungen dar. Sie folgen der Formel **(IV),** 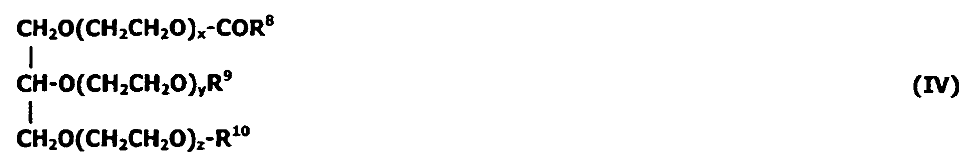 in der R⁸CO für einen linearen und verzweigten, gesättigten und ungesättigten Acylrest mit 6 bis 22 Kohlenstoffatomen, R⁹ und R¹⁰ unabhängig voneinander für Wasserstoff oder R³CO, x, y und z in Summe für 0 oder für Zahlen von 1 bis 30. Typische Beispiele sind Laurinsäuremonoglycerid, Ölsäuremonoglycerid, Laurinsäurediglycerid, Kokosfettsäuremonoglycerid, Kokosfettsäuretriglycerid, Palmitinsäuremonoglycerid, Palmitinsäuretriglycerid, Stearinsäuremonoglycerid, Stearinsäurediglycerid, Talgfettsäuremonoglycerid, Talgfettsäurediglycerid, Rizinusöl und hydriertes Rizinusöl, Behensäuremonoglycerid, Behensäurediglycerid sowie deren technische Gemische, die untergeordnet aus dem Herstellungsprozeß noch geringe Mengen an Triglycerid enthalten können.

Als dritte Gruppe von Wachskörpern kommen **Fettalkoholethoxylate**, die der Formel **(V)** folgen in den Wachsmischungen vor,

**R**^{**11**}**O(CH**_{**2**}**-CH**_{**2**}**-O)-H** **(V)**

in der R¹¹ für einen linearen, gegebenenfalls hydroxysubstituierten Alkylrest mit 16 bis 48, vorzugsweise 18 bis 36 Kohlenstoffatomen steht. Typische Beispiele für geeignete Alkohole sind Cetearylalkohol, Hydroxystearylalkohol, Behenylalkohol sowie Oxidationsprodukte langkettiger Paraffin. Insbesondere ethoxylierte Behenylalkohole werden als Wachskörper bevorzugt.

### Kationische Polymere

Weitere Hilfsstoffe in den Imprägnierlösungen sind kationische Polymere, die in den Imprägnierlösungen für die erfindungsgemäßen Kosmetiktücher in Mengen von 0,02 bis 3 Gew. %, vorzugsweise 0,05 bis 1,5 Gew.% und besonders bevorzugt in Mengen von 0,1 bis 1 Gew. % eingesetzt werden. Dazu zählen beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.
In den erfindungsgemäßen Zubereitungen werden bevorzugt kationisches Guar-Gum und/oder quaternierte Ammoniumsalz-Polymere eingesetzt.

### Polyole

Polyole, die im Sinne der Erfindung als fakultativer Bestandteil in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest, wie beispiels-weise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.
Bevorzugt sind Glycerin und Propylenglycol in den Mengen von 0 bis 20 Gew. %, bevorzugt 1 bis 15 Gew.% und besonders bevorzugt 3 bis 12 Gew.% bezogen auf die Imprägnierlösungen.

### Teilchengrößen der Wachsdispersionen und Teilchengrößenbestimmung

Die Teilchengrößen der Wachsdispersionen, die in den Imprägnier- und Coatinglösungen eingesetzt werden, haben einen wesentlichen Einfluß auf die sensorischen Eigenschaften und Schaum- und Reinigungseigenschaften der Tücher. Sie müssen für die optimale Einstellung durchschnittliche Teilchengrößen in den Dispersionen von maximal 13 µm , vorzugsweise maximal 4 µm und besonders bevorzugt maximal 2 µm betragen. Es werden dazu die herkömmlichen dem Fachmann bekannten Verfahren zur Dispersionsherstellung gewählt. So können die Partikelgrößen beispielsweise durch unterschiedliche Belastung der Dispersionen in Hochdruckhomogenisatoren eingestellt werden.
Die jeweiligen Bedingungen zur Einstellung der durchschnittlichen Teilchengrößen müssen jedoch je nach den gewählten Wachskörpern und weiteren Hilfsstoffen der Wachsdispersionen angepaßt werden. Da die Partikelverkleinerung in der Regel mit einer Temperaturerhöhung des Mediums einhergeht, und die Weiterverarbeitung der Wachsdispersionen ohne eine Veränderung der Partikelgrößen möglich sein soll, sind Wachskörper zu wählen, die einen Schmelzpunkt resp. Schmelzbereich von 55 bis 90 °C aufweisen.

Die Teilchengrößen wurden mit dem Laserpartikelanalysator Coulter LS 230 der Firma Beckman Coulter bestimmt. Das Gerät verwendet neben der klassischen Laserbeugung zusätzlich die PIDS (polarized intensity differential scattering) -Methode zur Erweiterung des Messbereiches zum Submikronbereich hin. Der Laser erzeugt Licht der Wellenlänge 750 nm, das durch die PIDS-Technologie um die Wellenlängen 450, 600 und 900 nm ergänzt wird. Die 132 Detektoren ergeben einen Gesamtmessbereich von 0,04 - 2000 µm, der in 116 Größenklassen unterteilt wird. Die Auswertung erfolgt durch die Software wahlweise nach der Frauenhofer sehen Beugungstheorie oder nach der Streulichttheorie nach Mie.

Die vermessenen Proben wurden durch das Probenzuführmodul um den Faktor 100 verdünnt, wobei VE-Wasser als Dispergiermedium diente. Das Probenzuführmodul gewährleistet eine ständige Zirkulation der dispergierten Probe durch die Messzelle. Vor Beginn der eigentlichen Messung wurde die zirkulierende Probe einer Ultraschallbehandlung von 30 s ausgesetzt. Dies erfolgte durch ein an das Messgerät angeschlossenes Ultraschall-Modul der Firma Beckman Coulter. Die Auswertung erfolgte ausschließlich nach der Streulichttheorie nach Mie bei Verwendung eines Brechungsindex von 1,47 für das dispergierte Medium. Die angegebenen durchschnittlichen Teilchengrößenangaben beziehen sich auf den D₅₀-Wert der Volumenverteilung.

### Herstellungsverfahren

Die Imprägnierlösungen mit pflegenden, feindispergierten partikulären Wachsdispersionen definierter Teilchengröße werden so auf das strukturierte Flächengebilde aufgetragen, dass die Dispersion nach dem gegebenenfalls nachfolgenden Trocknungsverfahren durch Warmlufttrocknung, Vakuumtrocknung oder Walzentrocknung als diskontinuierliche Schicht lose auf dem Träger haftet. Die Temperatur während des Trocknungsverfahrens ist so zu wählen, dass die Gewebetemperatur unterhalb dem Schmelzpunkt der Wachskörper bleibt, um ein Verschmelzen der Partikel mit dem Gewebe zu verhindern und zu ermöglichen, dass die Partikel nach Anfeuchten und mechanischer Bearbeitung der Tücher feindispergiert im generierten Schaum vorliegen. Dieses setzt auch voraus, dass die Vertiefungen im Gewebe und die Partikelgrößen der Wachskörperpartikel aufeinander abgestimmt sind. Die Grübchen im Gewebe dürfen nur kleiner oder deutlich größer als die dispergierten und danach aufgetragenen Wachskörperpartikel sein, da ein einfaches Dispergieren der Partikel während der Schaumbildung sonst nicht gewährleistet ist.

Die hergestellten imprägnierten und getrockneten Kosmetiktücher fassen sich bei Berührung trocken an. Dabei beträgt der Restwassergehalt nach der Trocknung, beziehungsweise Wassergehalt der erfindungsgemäßen trockenen Kosmetiktücher zwischen 0,1 und 4 Gew. %, vorzugsweise 0,5 bis 3 Gew. % und besonders bevorzugt zwischen 0,8 und 2 Gew. % Wasser - bezogen auf das Gewicht des trockenen nicht imprägnierten Trägerstoffes.

### Gewerbliche Anwendbarkeit

Vorgeschlagen werden Zubereitungen zur Imprägnierung von Kosmetiktüchern, **dadurch gekennzeichnet, dass** sie
a) eine Emulgatormischung, enthaltend nichtionische und amphotere Tenside im Mengenverhältnis 10 : 1 bis 1 : 1 bezogen auf die Menge der Emulgatoren und
b) eine Mischung aus Wachskörpern, enthaltend Wachsester, Partialglyceride und Fettallkoholethoxylate sowie c) mindestens ein Kationpolymer
enthalten.

In der Regel enthalten die Imprägnierlösungen
a) eine Emulgatormischung, enthaltend nichtionische und amphotere Tenside im Mengenverhältnis 5 : 1 bis 1,5 : 1 bezogen auf die Menge der Emulgatoren und
b) eine Mischung aus Wachskörpern, enthaltend Wachsester, Partialglyceride und Fettallkoholethoxylate, in der die durchschnittliche Partikelgröße der Wachspartikel maximal 13 µm beträgt sowie
c) mindestens ein Kationpolymer

Bevorzugt enthalten sie
a) eine Emulgatormischung, enthaltend nichtionische und amphotere Tenside im Mengenverhältnis 4 : 1 bis 2 : 1 bezogen auf die Menge der Emulgatoren und
b) eine Mischung aus Wachskörpern, enthaltend Wachsester, Partialglyceride und Fettallkoholethoxylate, in der die durchschnittliche Partikelgröße der Wachspartikel maximal 4 µm beträgt, sowie
c) mindestens ein Kationpolymer,
d) Polyole, sowie
e) anionische Tenside

Besonders bevorzugt enthalten sie
a) eine Emulgatormischung, enthaltend nichtionische und amphotere Tenside im Mengenverhältnis 4 : 1 bis 2 : 1 bezogen auf die Menge der Emulgatoren und
b) eine Mischung aus Wachskörpern, enthaltend Wachsester, Partialglyceride und Fettallkoholethoxylate, in der die durchschnittliche Partikelgröße der Wachspartikel maximal 2 µm beträgt sowie
c) mindestens ein Kationpolymer, und
d) Polyole
eingesetzt.

**Tabelle 1a: Zusammensetzung der Imprägnierlösungen - Mengenangaben in Gew.% Aktivsubstanz**

| **Komponente** | **üblich** | **bevorzugt** | **besonders bevorzugt** |
|---|---|---|---|
| Wachsester | 0,05 bis 10 | 0,5 bis 8 | 1,5 bis 6 |
| Fettalkoholethoxylate | 0,05 bis 3,0 | 0,2 bis 2 | 0,4 bis 1,5 |
| Partialglyceride | 0,01 bis 5 | 0,05 bis 3 4 | 0,5 bis 3 |
| Nichtionische Tenside | 1 bis 50 | 10 bis 40 | 20 bis 30 |
| Amphotenside, Betain | 0,5 bis 25 | 2 bis 15 | 4 bis 10 |
| Polyole | 0 bis 20 | 1 bis 15 | 3 bis 10 |
| Kationpolymere | 0,02 bis 3 | 0,05 bis 2,0 | 0,1 bis 1,5 |
| Wasser | ad 100,0 | | |

Die Zubereitungen für die Imprägnierlösungen können auch als Konzentrate eingesetzt werden

**Tabelle 1b: Konzentrate für den Einsatz in Imprägnierlösungen - Mengenangaben in Gew.% Aktivsubstanz**

| **Handelsname** | **INCI-Name** | **üblich** | **bevorzugt** | **besonders bevorzugt** |
|---|---|---|---|---|
| 1) Cutina CP | Cetylpalmitate | 0,1 bis 10 | 1 bis 8 | 3 bis 6 |
| 2) Eumulgin B 10 | Beheneth-10 | 0,1 bis 5 | 0,5 bis 2 | 0,8 bis 1,4 |
| 3) Cutina HR | Hydrogenated castor oil | 0 bis 5 | 0,1 bis 2 | 0,3 bis 0,8 |
| 4) Cutina GMS | Glycerylpalmitate | 0,01 bis 5 | 0,1 bis 2 | 0,3 bis 0,6 |
| 5) Monomuls 90- 018 | Glyceryloleate | 0,1 bis 5 | 1 bis 3 | 1,6 bis 2,2 |
| 6) Plantacare 2000 UP | Decyl Glucoside | 1 bis 50 | 5 bis 40 | 20 bis 25 |
| 7) Dehyton K | Cocamidopropyl Betaine | 1 bis 30 | 3 bis 15 | 5 bis 8 |
| 8) Plantacare 818 UP | Coco-Glucoside | 0,1 bis 20 | 1 bis 10 | 1,8 bis 6 |
| 9) Citronensäure | Citric acid | ad pH 6 | ad pH 6 | ad pH 6 |
| 10) Glycerin | Glycerine | 0 bis 20 | 3 bis 15 | 8 bis 10 |
| 11) Cosmedia Guar C 261 12) Konserviertes Wasser | Guar Hydroxypropyl Trimonium Chloride | 0,1 bis 3 | 0,5 bis 2 | 0,8 bis 1,4 |
| | | ad 100,0 | | |

### Herstellung:

Die Komponenten 1 bis 4 sowie die Komponenten 6 bis 8 und 12 werden auf 80 bis 85°C erwärmt bzw. geschmolzen, zusammengemischt und kaltgerührt. Komponente 5 wird der Emulsion bei 40 bis 50°C zugefügt. Komponenten 10 und 11 werden vorgemischt und der Emulsion nach dem Erkalten auf Raumtemperatur unter Rühren zugefügt. 0,1%ige Citronensäurelösung (9) wird der zubereitung bis zur pH-Einstellung von pH 5, 2 bis 6,8 zugefügt.

Die Konzentrate können mit Wasser und weiteren Hilfsstoffen eingesetzt werden, wobei der Anteil der Konzentrate in den fertigen Imprägnierlösungen bei 1 bis 99 Gew.%, vorzugsweise 10 bis 90 Gew.% und besonders bevorzugt 40 bis 60 Gew. % bezogen auf die Imprägnierlösung beträgt.

Die zur Imprägnierung eingesetzten Lösungen und/oder Dispersionen können ferner als weitere Hilfs- und Zusatzstoffe Ölkörper, Emulgatoren, Weichmacher, Rückfetter, Polymere, Siliconverbindungen, Lecithine, Phospholipide, biogene Wirkstoffe, UV-Lichtschutzfaktoren, Antioxidantien, Deodorantien, Antitranspirantien, Antischuppenmittel, Filmbildner, Hydrotrope, Solubilisatoren, Konservierungsmittel, Parfümöle, Farbstoffe und dergleichen enthalten.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃₋Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, I-sostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. **DE 19756377 A1**), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Weitere Emulgatoren

Als Emulgatoren kommen beispielsweise weitere nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

### ➢ Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18-}Fettsäuremono- und -diester von Anlagerungsprodukten von Ethylenoxid an Glycerin sind aus **DE 2024051 PS** als Rückfettungsmittel für kosmetische Zubereitungen bekannt.

### Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### ➢ Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### ➢ Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### ➢ Amphothere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropytdimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropion-säuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquatemierte Difettsäuretriethanolaminester-Salze, besonders bevorzugt sind.

### Polymere

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobomylacrylat-Copolymere, Methylvinylether/Maleinsäureanhydrid-Copolymere und deren Ester, unvemetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosm.Toil**. **108, 95 (1993)** aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der **EP 0693471 B1** beschrieben;
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-isopropylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der **EP 0694521 B1** beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzophenon-5-sulfonsäure und ihre Salze;
➢Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethylhexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtem wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** sowie **Parf.Kosm. 3, 11 (1999)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

### ➢ Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

### ➢ Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Znkglycinat.

### ➢ Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Ctronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

### ➢ Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe,
➢ Ölkomponenten,
➢ nichtionische Emulgatoren,
➢ Coemulgatoren,
➢ Konsistenzgeber,
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-nium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

### Insekten-Repellentien

Als Insekten-Repellentien kommen N,N-Diethyl-m-toluamid, 1,2-Pentandiol oder Ethyl Butylacetylaminopropionate in Frage

### Hydrotrope

Zur Verbesserung des Fließverhaltens bei der Auftragung können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedem-, Rosenholz), Kräutern und Gräsern (Estragon, Lemongras, Salbei, Thymian), Nadeln und Zweigen (Fchte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcydohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fxolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Stemanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

### Beispiele

Verschiedene Imprägnierdispersionen (Tabelle 3, 4) wurden durch einfaches Vermischen der Komponenten hergestellt; anschließend wurden Viskose/Polyester-Gewebe in einer Größe von 18,8 cm mal 14,8 cm entsprechend der Spezifikation (Tabelle 2) mit je 1-3,0 g der Dispersionen befeuchtet und bei max. 50°C über 2 h im Ofen getrocknet.

**Tabelle 2 Spezifikation für eingesetzten Trägerstoff für Kosmetiktücher**

| Test | Einheit | Grenzwert |
|---|---|---|
| Gewicht | g/m² | 80 +/- 8.0 |
| Zusammensetzung | % | 67 % Viscose |
| | | 33 % Polyester |
| Dicke | mm | 0.62 +/- 0.06 |
| Reißfestigkeit MD* | N/50mm | 130 - 160 |
| Reißfestigkeit TD** | N/50mm | 17 -21 |
| Dehnbarkeit MD | % | 21 +/- 8.0 |
| Dehnbarkeit TD | % | 116 +/- 25 |
| Reißfestigkeit feucht MD | N/50mm | 90 - 120 |
| Reißfestigkeit feucht TD | N/50mm | 15 - 19 |
| Aufsauggeschwindigkeit | secs | 3.0 max |
| Aufnahmekapazität | g/g | 5.5 - 7.5 |
| Dochtwirkung MD | mm/2mins | 100 +/- 20 |
| PH | | 7.0 +/- 1.0 |
| Trocknungsrest | % | 8.0 max |
| Fluorescence | | entspricht |
| Wasser lösl. Subst. | % | 0.5 max |
| Ether lösl. Sunst. | % | 0.5 max |
| Sulfatasche | % | 1.7 max |

| | | |
|---|---|---|
| * MD = Machine Direction - in Laufrichtung im Herstellungsprozeß | | |
| **TD = Transverse Direction - quer zur Laufrichtung im Herstellungsprozeß | | |

### Anwendungstest

Verschiedene Imprägnierlösungen/dispersionen (Mengenangaben in Gew.% Aktivsubstanz, Tabelle 3) wurden durch einfaches Vermischen der Komponenten hergestellt; anschließend wurden 67%Viskose/33%Polyester-Gewebe von 80 g/m² in einer Größe von 18,8 cm mal 14,8 cm entsprechend der Spezifikation (Tabelle 2) mit je 1-3,0 g der Dispersionen (Tabelle 3, 4) befeuchtet und bei max. 50°C über 2 Std. im Ofen getrocknet.
In einem Paneltest mit 4 Personen wurden die trockenen Kosmetiktücher zur Anwendung mit einem Überschuss an Wasser unter dem Wasserhahn befeuchtet, zwischen den Händen 20 s gerieben und zur Reinigung der Handoberflächen eingesetzt.
Beurteilt wurden das sensorische Gefühl des trockenen und des angefeuchteten Tuches in den Händen, das Hautgefühl während der Anwendung (feucht), das Hautgefühl nach Anwendung und Trocknung des Handrückens (trocken), die Zeit zur Schaumherstellung, die Struktur des Schaumes und das Schaumvolumen.
[Beurteilung: 1 = schlecht, 2 = mittelmäßig, 3 = gut]

**Tabelle 3 : Vergleich der Tücher mit Imprägnierlösung mit/ohne Wachsdispersionen**

| **Handelsname** | **INCI-Name** | **V1** | **1** |
|---|---|---|---|
| Plantacare 2000 UP | Decyl Glucoside | 20 | 20 |
| Dehyton K | Cocamidopropyl Betaine | 9 | 9 |
| Lamesoft PO 65 * mikrokristallin, < 0,5 | Coco-Glucoside, Glycerly Oleate | 3 | 3 |
| Lamesoft PW 45 * 0,5 - 1,5 µm | Cetyl Palmitate, Beheneth-10, Hydrogenated Castor Oil, Glyceryl Stearate | 0 | 7 |
| Glycerin | Glycerin | 4 | 4 |
| Cosmedia Guar C 261 | Guar Hydroxypropyl Trimonium Chloride | 0,5 | 0,5 |
| Wasser | | ad 100,0 | |
| Sensorik trockenes Tuch | Schlecht = 1 | 2222 | 2222 |
| | Mittelmäßig = 2 | | |
| | Gut = 3 | | |
| Sensorik feuchtes Tuch | Schlecht = 1 | 2211 | 3333 |
| | Mittelmäßig = 2 | | |
| | Gut = 3 | | |
| Hautgefühl während Anwendung (feucht) | 1 = schleimig | 2121 | 3333 |
| | 3 = Cremig sanft | | |
| Hautgefühl trockener Haut nach Anwendung | 1 = klebrig | 2112 | 2333 |
| | 3 = Seidig weich | | |
| Benötigte Zeit zur Schaumherstellung | > 15 s = 1 | 2222 | 2233 |
| | < 10 s = 3 | | |
| Schaumstruktur | Lose, grobporig = 1 | 1111 | 3333 |
| | Fest, feinporig = 3 | | |
| Schaumvolumen | Niedrig = 1 Hoch = 3 | 2222 | 2222 |
| | | | |

**Tabelle 4a: Rezepturbeispiele mit unterschiedlichen Imprägnierlösungen, Mengenangaben in Gew.% Aktivsubstanz**

| **Handelsname** | **INCI-Name** | **1** | **2** | **3** | **4** | **5** | **6** | **7** |
|---|---|---|---|---|---|---|---|---|
| Plantacare 2000 UP | Decyl Glucoside | 23 | 20 | 20 | 20 | 20 | 20 | 20 |
| Dehyton K | Cocamidopropyl Betaine | 10 | 10 | 3 | 10 | 3 | 10 | 3 |
| Lamesoft PO 65 * mikrokristallin, < 0,5 Lamesoft PW 45 * 0,5 - 1,5 µm | Coco-Glucoside, Glyceryl Oleate | 3 | 3 | 3 | 2 | 2 | 8 | 8 |
| | Cetyl Palmitate, Beheneth-10, Hydrogenated Castor Oil, Glyceryl Stearate | 7 | 7 | 7 | 8 | 8 | 2 | 2 |
| Plantacare 818 UP | Coco-Glucoside | 2 | - | - | - | - | - | - |
| Citronensäure | Citric acid | ad pH 6 | ad pH 6 | ad pH 6 | ad pH 6 | ad pH 6 | ad pH 6 | ad pH 6 |
| Glycerin | Glycerin | 5 | 3 | - | 3 | - | 3 | - |
| Cosmedia Guar C 261 | Guar Hydroxypropyl Trimonium Chloride | 0,5 | 0,5 | 0,5 | 0,5 | 0,5 | 0,5 | 0,5 |
| Wasser | | Ad 100,0 | | | | | | |

**Tabelle 4b: Rezepturbeispiele mit unterschiedlichen Imprägnierlösungen, Mengenangaben in Gew.% Aktivsubstanz**

| **Handelsname** | **INCI-Name** | **8** | **9** | **10** | **11** | **12** | **13** | **14** |
|---|---|---|---|---|---|---|---|---|
| Plantacare 2000 UP | Decyl Glucoside | 23 | 20 | 20 | 20 | 20 | 20 | 20 |
| Dehyton K | Cocamidopropyl Betaine | 10 | 10 | 3 | 10 | 3 | 10 | 3 |
| Lamesoft PO 65 * mikrokristallin, < 0,5 Lamesoft PW 45 * 0,5 - 1,5 µm | Coco-Glucoside, Glyceryl Oleate | 5 | 5 | 5 | 1 | 10 | 12 | 1 |
| | Cetyl Palmitate, Beheneth-10, Hydrogenated Castor Oil, Glyceryl Stearate | 5 | 5 | 5 | 10 | 1 | 1 | 12 |
| Plantacare 818 UP | Coco-Glucoside | 2 | - | - | - | - | - | - |
| Citronensäure | Citric acid | ad pH 6 | ad pH 6 | ad pH 6 | ad pH 6 | ad pH 6 | ad pH 6 | ad pH 6 |
| Glycerin | Glycerin | 5 | 3 | - | 3 | - | 3 | - |
| Cosmedia Guar C 261 | Guar Hydroxypropyl Trimonium Chloride | 0,5 | 0,5 | 0,5 | 0,5 | 0,5 | 0,5 | 0,5 |
| Wasser | | ad 100,0 | | | | | | |

## Patentansprüche

1. Zubereitungen zur Imprägnierung von Kosmetiktüchern, **dadurch gekennzeichnet, dass** sie
a) eine Emulgatormischung, enthaltend nichtionische und amphotere Tenside im Mengenverhältnis 10 : 1 bis 1 : 1 bezogen auf die Menge der Emulgatoren und
b) eine Mischung aus Wachskörpern, enthaltend Wachsester, Partialglyceride und Fettalkoholethoxylate sowie
c) mindestens ein Kationpolymer
enthalten.

2. Zubereitungen zur Imprägnierung von Kosmetiktüchem nach Anspruch 1, **dadurch gekennzeichnet, dass** die durchschnittlichen Partikelgrößen der Wachskörper (Komponente b) eine Größe von maximal 13 µm aufweisen.

3. Zubereitungen zur Imprägnierung von Kosmetiktüchern nach den Ansprüchen 1 und/oder 2, **dadurch gekennzeichnet, dass** sie als nichtionische Tenside Alkyloligoglycoside und/oder Fettalkoholethoxylate enthalten.

4. Zubereitungen zur Imprägnierung von Kosmetiktüchern nach mindestens einem der Ansprüche 1 bis 3, **dadurch gekennzeichnet, dass** sie als amphotere Tenside Betaine enthalten.

5. Zubereitungen zur Imprägnierung von Kosmetiktüchem nach mindestens einem der Ansprüche 1 bis 4, **dadurch gekennzeichnet, dass** sie als Wachsester Cetylpalmitat enthalten.

6. Zubereitungen zur Imprägnierung von Kosmetiktüchem nach mindestens einem der Ansprüche 1 bis 5, **dadurch gekennzeichnet, dass** sie als Partialglyceride Glycerinmono- und/oder dioleat und/oder Glycerinmono-und/oder distearat und/oder hydriertes Rizinusöl enthalten.

7. Zubereitungen zur Imprägnierung von Kosmetiktüchern nach mindestens einem der Ansprüche 1 bis 6, **dadurch gekennzeichnet, dass** das Kationpolymer ausgewählt ist aus der Gruppe, die gebildet wird von kationischen Cellulosederivaten, kationischer Stärke, Copolymeren von Diallylammoniumsalzen und Acrylamiden, quatemierten Vinylpyrrolidon/Vinylimidazol-Polymeren, Kondensationsprodukten von Polyglycolen und Aminen, quaternierte Kollagenpolypeptide, quatemierten Weizenpolypeptiden, Polyethyleniminen, kationischen Siliconpolymeren, wie z.B. Amodimethiconen, Copolymeren der Adipinsäure und Dimethylaminohydroxypropyldiethylentriamin (Cartaretine®/Sandoz), Copolymeren der Acrylsäure mit Dimethyl-diallylammoniumchlorid (Merquat® 550/Chemviron), Polyaminopolyamiden, kationischen Chitinderivaten Kondensationsprodukten aus Dihalogenalkylen, kationischem Guar-Gum, quatemierten Ammoniumsalz-Polymeren.

8. Zubereitungen zur Imprägnierung von Kosmetiktüchern nach mindestens einem der Ansprüche 1 bis 7, **dadurch gekennzeichnet, dass** sie als Kationpolymer kationisches Guar-Gum und/oder quaternierte Ammoniumsalz-Polymere enthalten.

9. Zubereitungen zur Imprägnierung von Kosmetiktüchem nach mindestens einem der Ansprüche 1 bis 8, **dadurch gekennzeichnet, dass** sie als fakultative Komponente d) Polyole enthalten.

10. Zubereitungen zur Imprägnierung von Kosmetiktüchem nach mindestens einem der Ansprüche 1 bis 9, **dadurch gekennzeichnet, dass** sie als fakultative Komponente e) anionische Tenside enthalten.

11. Verfahren zur Herstellung von Kosmetiktüchem **dadurch gekennzeichnet, dass** man ein Gewebe mit einer Imprägnierlösung nach mindestens einem der Ansprüche 1 bis 9, befeuchtet und gegebenenfalls nachfolgend das Lösungsmittel bis auf einen Restgehalt von 0.1 bis 4 Gew.% - bezogen auf das Gewicht des Kosmetiktuches - heraustrocknet.

## Claims

1. Preparations for impregnating cosmetic wipes, **characterized in that** they contain
(a) an emulsifier mixture containing nonionic and amphoteric surfactants in a quantity ratio of 10:1 to 1:1, based on the quantity of emulsifiers,
(b) a mixture of wax components containing wax esters, partial glycerides and fatty alcohol ethoxylates and
(c) at least one cationic polymer.

2. Preparations for impregnating cosmetic wipes as claimed in claim 1, **characterized in that** the mean particle sizes of the wax components (component b) are at most 13 µm.

3. Preparations for impregnating cosmetic wipes as claimed in claims 1 and/or 2, **characterized in that** they contain alkyl oligoglycosides and/or fatty alcohol ethoxylates as nonionic surfactants.

4. Preparations for impregnating cosmetic wipes as claimed in at least one of claims 1 to 3, **characterized in that** they contain betaines as amphoteric surfactants.

5. Preparations for impregnating cosmetic wipes as claimed in at least one of claims 1 to 4, **characterized in that** they contain cetyl palmitate as the wax ester.

6. Preparations for impregnating cosmetic wipes as claimed in at least one of claims 1 to 5, **characterized in that** they contain glycerol mono- and/or dioleate and/or glycerol mono- and/or distearate and/or hydrogenated castor oil as the partial glycerides.

7. Preparations for impregnating cosmetic wipes as claimed in at least one of claims 1 to 6, **characterized in that** the cationic polymer is selected from the group consisting of cationic cellulose derivatives, cationic starch, copolymers of diallyl ammonium salts and acrylamides, quaternized vinyl pyrrolidone/vinyl imidazole polymers, condensation products of polyglycols and amines, quaternized collagen polypeptides, quaternized wheat polypeptides, polyethyleneimines, cationic silicone polymers such as, for example, amodimethicone, copolymers of adipic acid, and dimethylaminohydroxypropyl diethylenetriamine, copolymers of acrylic acid with dimethyl diallyl ammonium chloride, polyaminopolyamides, cationic chitin derivatives, condensation products of dihaloalkyls, cationic guar gum, quaternized ammonium salt polymers.

8. Preparations for impregnating cosmetic wipes as claimed in at least one of claims 1 to 7, **characterized in that** they contain cationic guar gum and/or quaternized ammonium salt polymers as the cationic polymer.

9. Preparations for impregnating cosmetic wipes as claimed in at least one of claims 1 to 8, **characterized in that** they contain polyols as an optional component d).

10. Preparations for impregnating cosmetic wipes as claimed in at least one of claims 1 to 9, **characterized in that** they contain anionic surfactants as an optional component e).

11. A process for the production of cosmetic wipes, **characterized in that** a cloth is moistened with the impregnating solution claimed in at least one of claims 1 to 9 and the solvent is then optionally removed by drying to a residual content of 0.1 to 4% by weight, based on the weight of the cosmetic wipe.

## Revendications

1. Préparations pour l'imprégnation de lingettes cosmétiques,
**caractérisées en ce qu'**
elles contiennent
a) un mélange émulsifiant contenant des tensioactifs non ioniques et des tensioactifs amphotères dans un rapport quantitatif de 10:1 à 1:1 par rapport à la quantité d'émulsifiants,
b) un mélange de corps cireux contenant des esters de cire, des glycérides partiels et des éthoxylates d'alcools gras, ainsi que
c) au moins un polymère cationique.

2. Préparations pour l'imprégnation de lingettes cosmétiques selon la revendication 1,
**caractérisées en ce que**
la taille moyenne des particules des corps cireux (composant b) est de 13 µm maximum.

3. Préparations pour l'imprégnation de lingettes cosmétiques selon les revendications 1 et/ou 2,
**caractérisées en ce qu'**
elles contiennent des oligolycosides d'alkyle et/ou des éthoxylates d'alcool gras en tant que tensioactifs non ioniques.

4. Préparations pour l'imprégnation de lingettes cosmétiques selon au moins l'une des revendications 1 à 3,
**caractérisées en ce qu'**
elles contiennent des bétaïnes en tant que tensioactifs amphotères.

5. Préparations pour l'imprégnation de lingettes cosmétiques selon au moins l'une des revendications 1 à 4,
**caractérisées en ce qu'**
elles contiennent du palmitate de cétyle en tant qu'ester de cire.

6. Préparations pour l'imprégnation de lingettes cosmétiques selon au moins l'une des revendications 1 à 5,
**caractérisées en ce qu'**
elles contiennent, en tant que glycérides partiels, du monooléate et/ou du dioléate de glycérol et/ou du monostéarate et/ou du distéarate de glycérol et/ou de l'huile de ricin hydrogénée.

7. Préparations pour l'imprégnation de lingettes cosmétiques selon au moins l'une des revendications 1 à 6,
**caractérisées en ce que**
le polymère cationique est choisi dans le groupe formé de dérivés cationiques de cellulose, d'amidon cationique, de copolymères de sels de diallylammonium et d'acrylamides, de polymères de vinylpyrrolidone/vinylimidazole quaternisés de produits de condensation de polyglycols et d'amines, de polypeptides de collagène quaternisés de polypeptides de froment quaternisés de polyéthylèneimines, de polymères de silicone cationiques comme, par exemple, les amodiméthicones, de copolymères d'acide adipique et de diméthylaminohydroxypropyldiéthylènetriamine (Cartaretine®/Sandoz), de copolymères d'acide acrylique avec du chlorure de diméthyl-diallylammonium (Merquat® 550/Chemviron), de polyaminopolyamides, de dérivés cationiques de chitine, de produits de condensation de dihalogénoalkyles, de gomme de guar cationique, de polymères de sel d'ammonium quaternisés.

8. Préparations pour l'imprégnation de lingettes cosmétiques selon au moins l'une des revendications 1 à 7,
**caractérisées en ce qu'**
elles contiennent, en tant que polymère cationique, de la gomme de guar cationique et/ou des polymères de sel d'ammonium quaternisés.

9. Préparations pour l'imprégnation de lingettes cosmétiques selon au moins l'une des revendications 1 à 8,
**caractérisées en ce qu'**
elles contiennent, en tant que composant d) facultatif, des polyols.

10. Préparations pour l'imprégnation de lingettes cosmétiques selon au moins l'une des revendications 1 à 9,
**caractérisées en ce qu'**
elles contiennent, en tant que composant e) facultatif, des tensioactifs anioniques.

11. Procédé de fabrication de lingettes cosmétiques,
**caractérisé en ce qu'**
on humidifie un tissu avec une solution d'imprégnation selon au moins l'une des revendications 1 à 9 et, le cas échéant, on élimine ensuite, le solvant par séchage jusqu'à une teneur résiduelle de 0,1 à 4 % en poids, - par rapport au poids total de la lingette cosmétique.
